# FASCICULE DE BREVET EUROPEEN

(11) **EP 3 668 963 B1**
(45) Date de publication et mention de la délivrance du brevet: **05.05.2021**
(21) Numéro de dépôt: 18753203.1
(22) Date de dépôt: 17.08.2018
(51) Int. Cl.: C12M 1/00, C12M 1/02

(54) **REACTEUR A DISPOSITIF D'ECLAIRAGE ET DE CHAUFFAGE INTEGRE**
REAKTOR MIT EINGEBAUTER BELEUCHTUNG UND HEIZVORRICHTUNG
REACTOR WITH BUILT-IN LIGHTING AND HEATING DEVICE

(30) Priorité: 18.08.2017 FR 1757745
(43) Date de publication de la demande: 24.06.2020
(73) Titulaire: Fermentalg, 33500 Libourne (FR)
(72) Inventeur: CAGNAC, Olivier, 33500 LIBOURNE (FR)
(74) Mandataire: Be IP
(86) Numéro de dépôt international: PCT/EP2018/072371
(87) Numéro de publication internationale: WO 2019/034792

(56) Documents cités:
- WO-A1-2014/174182
- JP-A- 2012 183 002
- KR-B1- 101 043 583

## Description

### DOMAINE DE L'INVENTION

La présente invention concerne le domaine technique général des réacteurs à éclairage intégré, notamment pour la culture de micro-organismes photosensibles.

Il peut s'agir d'un bioréacteur mais aussi d'un réacteur chimique ou physico-chimique

### ARRIERE PLAN DE L'INVENTION

La notion de bioréacteur, ou réacteur biologique, désigne ici un réacteur au sein duquel se développent des phénomènes biologiques, tels qu'une croissance de cultures de micro-organismes pures ou d'un consortium de micro-organismes (notamment des microalgues), dans des domaines très variés tels que le traitement d'effluents, la production de biomasse contenant des biomolécules d'intérêt (c'est-à-dire des biomolécules que l'on sait valoriser). Cette notion englobe donc notamment les réacteurs appelés fermenteurs.

Un réacteur comporte typiquement une cuve fermée dans laquelle est monté un élément de brassage ou d'agitation destiné à favoriser une homogénéisation du contenu de la cuve. Un tel élément de brassage est habituellement constitué d'un arbre vertical portant des pales ou des turbines dont le mouvement, au sein de la masse en cours de traitement dans le réacteur, assure le brassage et son homogénéité.

Divers types de conditions opératoires peuvent être nécessaires pour la croissance des espèces biologiques au sein d'un tel bioréacteur ou fermenteur ; on connaît ainsi, notamment, des régimes de croissance autotrophe (ou photo-autotrophe) avec un apport de lumière (on parle aussi de photosynthèse) ou de croissance mixotrophe (avec un apport combiné de source carbonée et de lumière). Il faut noter également que la lumière peut agir sur le métabolisme des cellules en induisant ou réprimant la production de certains composés, indépendamment de la croissance et de la photosynthèse. Un apport de lumière lors de la culture peut donc être utile même lorsque les micro-organismes sont hétérotrophes.

Diverses configurations ont déjà été proposées pour effectuer un tel apport de lumière, en continu ou de manière cyclique (avec des cycles dont la durée peut varier de quelques minutes à plusieurs heures), voire sous forme d'impulsions à la manière de flash, avec un spectre approchant de celui de la lumière du jour ou au contraire étroitement centré sur une longueur d'onde choisie.

Le document WO 2014/174182décrit un réacteur comportant une cuve destinée à contenir une masse à traiter et munie :
- d'un ensemble tournant autour d'un axe destiné à assurer un brassage de cette masse à traiter, et
- d'une pluralité de moyens d'illumination destinés à favoriser le traitement de cette masse, les moyens d'illumination étant intégrés dans des contre-pales rectangulaires fixées à une face interne de la paroi latérale de la cuve, et s'étendant dans des plans orientés vers l'axe de rotation de l'ensemble tournant.

Ainsi, les contre-pales fixées à la face interne de la paroi latérale de la cuve jouent une double fonction. Elles permettent :
- d'une part la formation d'un vortex au sein de la masse à traiter sous l'action de l'ensemble tournant, et
- d'autre part l'illumination la masse à traiter grâce aux moyens d'illumination qui y sont intégrés.

On a illustré à la figure 1 une vue en coupe transversale d'un exemple de contre-pale selon le document WO 2014/174182.

La contre-pale comprend :
- une plaque 10 sensiblement rectangulaire,
- une pluralité de moyens d'illumination 20 montés sur une face supérieure de la plaque 10,
- des câbles électriquement conducteurs 30 disposés dans un canal central 40 de la plaque 10, les câbles électriquement conducteurs 30 permettant de connecter électriquement les moyens d'illumination 20 à une source d'alimentation en énergie électrique (non représentée),
- une lame sensiblement transparente 50 (ayant des longueur et largeur identiques à la plaque) montée sur la face supérieure de la plaque 10 de sorte que les moyens d'illumination 20 s'étendent entre la lame 50 et la plaque 10,
- un châssis rectangulaire 60 (ayant des longueur et largeur identiques à la plaque) monté sur la lame rectangulaire 50, et
- des moyens de fixation 70 - tels que des boulons - pour fixer ensemble la plaque 10, la lame 40 et le châssis 60.

L'utilisation d'une telle contre-pale permet au réacteur décrit dans WO 2014/174182 de traiter efficacement une masse biologique nécessitant un apport de lumière pour favoriser sa croissance.

Les moyens d'illumination utilisés dans WO 2014/174182 sont des diodes électroluminescentes (ou « LED », acronyme de l'expression anglo-saxonne « *light-emitting diode* ») d'une puissance de 1 Watt. La quantité de lumière délivrée par ces moyens d'illumination peut donc être insuffisante dans le cadre de certains traitements.

En effet, la pénétration de la lumière dans le réacteur diminue en fonction de la densité cellulaire présente ; lorsque la densité cellulaire est importante, il peut donc s'avérer nécessaire d'éclairer fortement pour que les cellules se trouvant au centre du réacteur reçoivent de la lumière.

Par ailleurs, plus le diamètre d'un réacteur est important, plus l'intensité de la lumière générée par les moyens d'illumination doit être importante afin d'éclairer les cellules qui se trouvent au centre du réacteur.

Pour certaines applications, notamment industrielles, il serait donc souhaitable de disposer de moyens d'illumination plus puissants.

Toutefois, l'utilisation de moyens d'illumination plus puissants induit une augmentation de la consommation en énergie électrique. Or dans le cadre d'applications industrielles, une autre contrainte forte consiste à limiter les coûts associés à la culture de micro-organismes photosensibles.

Un but de la présente invention est de proposer un réacteur incluant un dispositif d'éclairage plus puissant que les contre-pales décrites dans WO 2014/174182, tout en limitant la consommation en énergie électrique du réacteur. Le document KR 101 043 583 décrit un réacteur avec une plaque munie des moyens d'illumination à base de LEDs pour réduire la génération de chaleur.

### BREVE DESCRIPTION DE L'INVENTION

A cet effet, l'invention propose un réacteur incluant une cuve destinée à contenir une masse à traiter et au moins un dispositif d'éclairage et de chauffage destiné à favoriser le traitement de cette masse, ***remarquable en ce que*** le dispositif d'éclairage et de chauffage comprend :
- une plaque incluant au moins une rainure s'étendant longitudinalement et composée d'un fond et de deux parois latérales,
- des moyens d'illumination pour générer un rayonnement lumineux, lesdits moyens d'illumination étant disposés dans la rainure et étant en contact thermique avec le fond de la rainure, de sorte que la chaleur générée par les moyens d'illumination est transmise à la masse à traiter par l'intermédiaire du fond de la plaque.

Ainsi dans le cadre de la présente invention, la chaleur dissipée par les moyens d'illumination est transmise à la masse à traiter : le dispositif d'éclairage permet donc également le chauffage de la masse contenue dans le réacteur.

Ceci est très avantageux, notamment dans le cas de la culture de *Galdieria* (ou d'autres souches dont la culture nécessite à la fois de la lumière et de la chaleur). Les souches de *Galdieria* ont en effet une exigence tant pour l'éclairement que pour les températures (supérieures à 35 °C) en ce qui concerne leurs conditions de croissance.

Des aspects préférés mais non limitatifs de l'ensemble selon l'invention sont les suivants :
- le dispositif d'éclairage et de chauffage peut comprendre :
   o au moins un câble électriquement conducteur pour connecter électriquement les moyens d'illumination à une source en alimentation électrique distante, le câble s'étendant le long d'une des parois latérales de la rainure,
   o au moins une lame transparente au rayonnement lumineux s'étendant sur la plaque pour coiffer la rainure ;
- les moyens d'illumination peuvent comprendre au moins un module de diodes électroluminescentes à montage direct de puces,
- le matériau constituant la lame peut être du verre,
- le dispositif d'éclairage et de chauffage peut comprendre en outre :
   o au moins un châssis de retenue s'étendant sur la lame,
   o des moyens de fixation destinés à coopérer avec la plaque, la lame et le châssis pour solidariser la plaque, la lame et le châssis ;
- le dispositif d'éclairage et de chauffage peut comprendre en outre :
   o au moins un premier joint en élastomère entre la lame et la plaque, et
   o au moins un deuxième joint en élastomère entre la lame et le châssis, les premier et deuxième joints s'étendant à la périphérie de la lame ;
- chaque plaque peut comprendre au moins deux panonceaux démontables reliés bord à bord de sorte à s'étendre dans un même plan pour constituer la plaque ;
- les moyens d'illumination d'un panonceau peuvent être activables indépendamment de l'activation des moyens d'illumination des autres panonceaux ;
- le réacteur peut également comprendre un agitateur tournant autour d'un axe Z pour brasser la masse à traiter, chaque dispositif d'éclairage et de chauffage étant fixé à une face interne de la cuve et s'étendant dans un plan orienté vers l'axe de l'agitateur pour former une contre-pâle permettant d'empêcher la formation d'un vortex au sein de la masse à traiter ;
- la dissipation de chaleur permet d'augmenter la température du milieu de culture d'au moins 2 degrés Celsius et facilite ainsi la croissance des microorganismes.

### BREVE DESCRIPTION DES DESSINS

D'autres avantages et caractéristiques de l'ensemble selon l'invention ressortiront mieux de la description qui va suivre de plusieurs variantes d'exécution, données à titre d'exemples non limitatifs, à partir des dessins annexés sur lesquels :
- La figure 1 est une vue en coupe transversale d'une contre-pale de l'art antérieur telle que décrite dans WO 2014/174182 ;
- Les figures 2 et 3 sont des vues en coupe transversale de deux modes de réalisation d'un dispositif d'éclairage et de chauffage selon l'invention,
- La figure 4 est une vue en coupe transversale d'un troisième mode de réalisation du dispositif d'éclairage et de chauffage,
- La figure 5 est une représentation schématique d'un exemple de réacteur incluant le dispositif d'éclairage et de chauffage,
- La figure 6 est une courbe illustrant les variations de température dans un réacteur en fonction des variations de puissance lumineuse du dispositif d'éclairage et de chauffage selon l'invention,
- La figure 7 est un graphique démontrant l'effet du dégagement de chaleur par des dispositifs d'éclairage et de chauffage selon l'invention lors de la croissance d'une souche de *Galderia sulphuraria.*

### DESCRIPTION DETAILLEE DE L'INVENTION

On va maintenant décrire différents exemples de dispositifs d'éclairage et de chauffage selon l'invention permettant :
- la génération d'un rayonnement lumineux plus puissant que les contre-pales décrites dans WO 2014/174182, et
- la génération de chaleur utilisée pour réchauffer la masse à traiter.

Dans ces différentes figures, les éléments équivalents sont désignés par la même référence numérique.

Comme décrit précédemment, même si les contre-pales selon WO 2014/174182 permettent de satisfaire au traitement de nombreux types de masse nécessitant un apport en lumière, il peut être nécessaire de disposer pour certaines applications d'une illumination plus puissante.

Les moyens d'illumination 20 de WO 2014/174182 consistent en des diodes électroluminescentes d'une puissance de 1 Watt.

Toutefois, le simple remplacement de diodes électroluminescentes de 1 Watt par des moyens d'illumination plus puissants n'est pas envisageable.

En effet, des moyens d'illumination plus puissants induisent la génération d'une plus forte chaleur. Cette chaleur, si elle n'est pas évacuée, peut endommager les moyens d'illumination.

Or, du fait de la conception de la contre-pale décrite dans WO 2014/174182, l'évacuation de cette chaleur n'est pas efficace. En effet, le canal central 40 de la plaque 10 (et plus précisément l'air contenu dans ce canal central 40) forme un isolant thermique empêchant l'évacuation de la chaleur produite par les moyens d'illumination, de sorte que celle-ci se concentre au niveau des moyens d'illumination et provoque leur dégradation prématurée. Pour pallier cet inconvénient, WO 2014/174182 propose d'ailleurs l'intégration de circuits d'évacuation de la chaleur dans l'épaisseur des contre-pales.

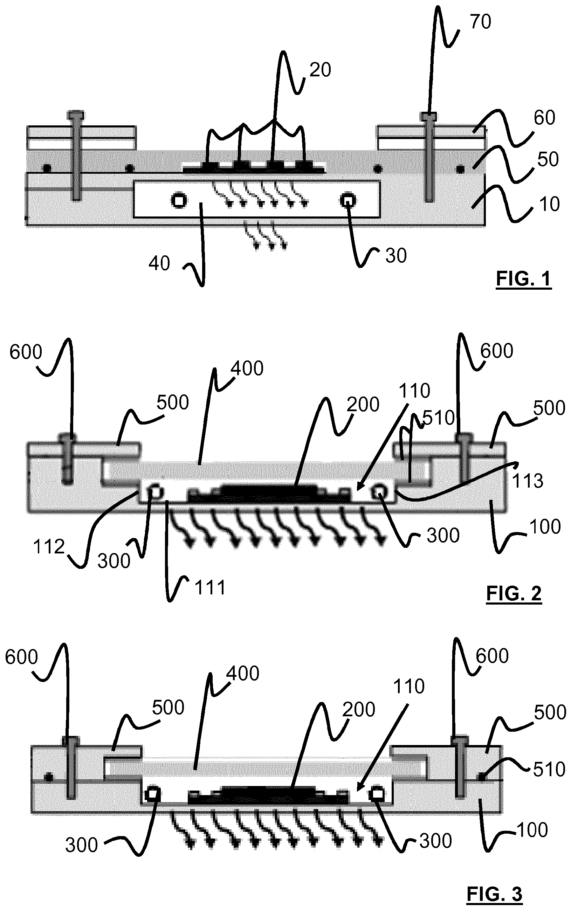

Les inventeurs de la présente invention ont proposé une autre approche en développant un nouveau dispositif permettant à la fois l'éclairage et le chauffage d'une masse à traiter. Un tel dispositif d'éclairage et de chauffage est illustré aux figures 2 à 4.

Ce dispositif d'éclairage et de chauffage est destiné à être intégré dans un réacteur pour le traitement d'une masse. Il est décrit ici en référence au traitement d'une biomasse formée de micro-organismes, par exemple des microalgues ; on comprend toutefois que la description qui suit s'applique également à d'autres types de réacteurs, chimiques ou physico-chimiques.

On va maintenant décrire plus en détails le dispositif d'éclairage et de chauffage en référence aux figures 2 à 7. Ce dispositif d'éclairage et de chauffage comprend une plaque 100, des moyens d'illumination 200, des câbles électriquement conducteurs 300, une lame sensiblement transparente 400, un châssis 500, et des moyens de fixation 600.

### 1. Dispositif d'éclairage et de chauffage

### 1.1. Plaque

La plaque 100 présente une forme rectangulaire. Elle est de préférence réalisée dans un matériau thermiquement conducteur peu sensible à la corrosion tel que de l'acier inoxydable.

La plaque 100 comprend une (ou plusieurs) rainure(s) 110 s'étendant longitudinalement. La rainure 110 est de préférence rectiligne et peut avoir toute forme connue de l'homme du métier en section transversale, telle qu'une forme en V ou en U. Chaque rainure 110 constitue un logement pour des moyens d'illumination 200 et des câbles électriquement conducteurs 300. La rainure 110 comprend un fond 111 et des parois latérales 112, 113. Avantageusement, la paroi constituant le fond 111 de la rainure 110 est pleine pour favoriser les échanges thermiques entre l'intérieur de la rainure 110 et l'extérieur de la plaque 100.

La plaque 100 comprend également des trous borgnes (éventuellement taraudés) s'étendant de part et d'autre de la rainure 110. Les trous borgnes permettent la réception de pas de vis de moyens de fixation 600 - tels que des boulons - pour fixer ensemble la plaque 100, la lame 400 et le châssis 500.

La plaque 100 forme un support pour les moyens d'illumination 200. Elle est avantageusement démontable vis-à-vis de la paroi de la cuve, donc extractible hors de la cuve. Un avantage est qu'ainsi, les opérations de maintenance de la plaque 100 et des moyens d'illumination 200, ainsi que celles de la cuve, sont simplifiées. Un autre avantage est qu'une même cuve peut être équipée de diverses configurations d'illumination (il suffit de disposer de plusieurs jeux de plaques comportant un nombre différent et/ou une nature différente de sources individuelles d'illumination). Le fait de disposer plusieurs jeux de plaque 100 comportant des configurations d'illumination différentes ou non permet en outre de minimiser les interruptions de fonctionnement lors des opérations de maintenance, puisqu'un jeu de plaques 100 peut être en maintenance pendant qu'un autre est en service ; de même, en cas de panne, la réparation peut se faire, de manière aisée, après s'être éventuellement contenté de remplacer les plaques 100 par d'autres plaques 100 munies d'une pluralité similaire de moyens d'illumination 200.

Chaque plaque 100 peut être composée d'un ou plusieurs panonceaux reliés bord à bord de sorte à s'étendre dans un même plan pour constituer la plaque 100. Le fait que chaque plaque 100 soit composée d'un assemblage de panonceaux permet de faciliter la manutention des plaques 100, notamment dans le cadre de cuves de grandes capacités (cuve de 1000 litres ou plus). Avantageusement, les moyens d'illumination 200 portés par chaque panonceau peuvent être activables séparément. Ceci permet d'activer uniquement les moyens d'illumination 200 submergés par la masse à traiter même si cette masse à traiter ne remplit pas toute la cuve.

### 1.2. Moyens d'illumination

Les moyens d'illumination 200 permettent de générer un éclairement favorisant le traitement de la masse à traiter. Ils peuvent être de tout type connu de l'homme du métier.

Les moyens d'illumination 200 peuvent par exemple consister en un module de diodes électroluminescentes à montage direct de puces (également connues sous le nom de LED « COB », acronyme de l'expression anglosaxonne « Chip On Board »). Un module de LED COB est composé de plusieurs puces LED fixées à un substrat - généralement de céramique. Un module de LED COB utilise un seul circuit avec seulement deux contacts pour alimenter les nombreuses puces de diode qui y sont installées. Le module de LED COB comportant plusieurs puces, la surface d'émission lumineuse est plus importante, ce qui permet d'obtenir une sortie lumineuse par centimètre carré beaucoup plus grande. Le fait que les moyens d'illumination 200 consistent en des modules de diodes électroluminescentes à montage direct de puces permet de générer un éclairement plus puissant et dense.

Les moyens d'illumination 200 sont par exemple LOHAS LED Chicago, WI 60601.

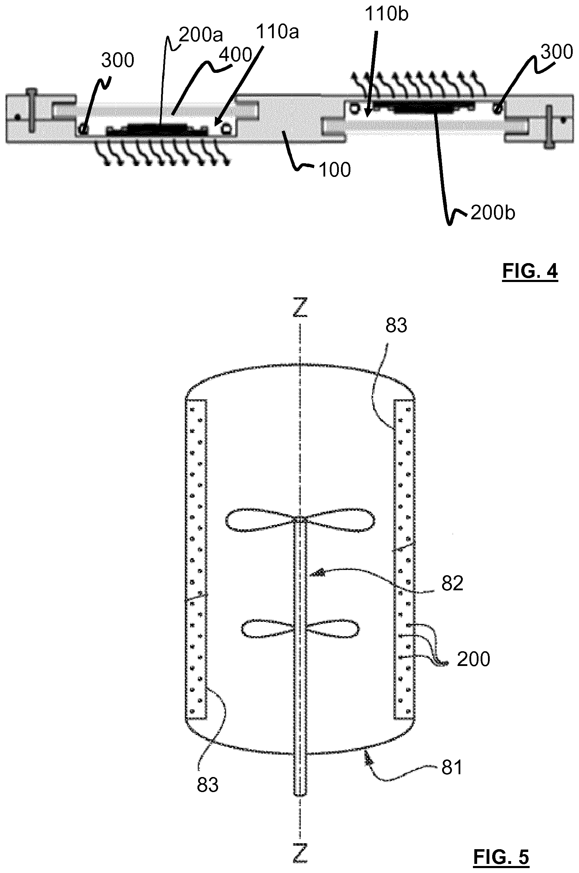

Les moyens d'illumination 200 peuvent être distribués sur une seule face de la plaque 100 (cf. figure 2 et 3) ou sur les deux faces de la plaque 100. Par exemple, en référence la figure 4, on a illustré une variante de réalisation dans laquelle la plaque 100 comprend :
- une première rainure 110a s'étendant longitudinalement sur l'une de ses faces et dans laquelle sont logés des premiers moyens d'illumination 200a,
- une deuxième rainure 110b s'étendant longitudinalement sur l'autre de ses faces et dans laquelle sont logés des deuxièmes moyens d'illumination 200b,
les première et deuxième rainures 110a, 110b étant décalées transversalement de sorte à s'étendre parallèlement sur l'une et l'autre des faces de la plaque 100.

Lorsque la plaque 100 comprend des moyens d'illumination 200a, 200b sur ses deux faces, les moyens d'illumination 200a sur une face de la plaque 100 peuvent être réparties en fonction de la répartition des moyens d'illumination 200b sur l'autre face, et s'étendre transversalement dans un même plan ou au contraire être décalés.

Les moyens d'illumination 200 peuvent être tous identiques en ayant un même régime d'excitation. En variante, les moyens d'illumination 200 peuvent être différents. Notamment, les moyens d'illumination 200 d'une plaque 100 peuvent avoir :
- des régimes d'excitation distincts (par exemple régime continu pour certains moyens d'illumination, et régime flash à une fréquence comprise entre 1 et 150 kHz pour d'autres moyens d'illumination), et/ou
- des spectres d'émission distincts (par exemple en lumière blanche pour certains moyens d'illumination et en lumière bleu pour d'autres), etc.

### 1.3. Câble électriquement conducteur

Le (ou les) câble(s) électriquement conducteur 300 permettent d'alimenter les moyens d'illumination 200 en énergie électrique. Avantageusement, les moyens d'illumination 200 peuvent être montés en série ou en parallèle.

Le câble 300 comprend des fils électriquement conducteurs destinés à être connectés aux bornes d'alimentation des moyens d'illumination 200 d'une part, et d'une source d'alimentation en énergie électrique (non représentée) d'autre part. Les fils électriques sont entourés d'un matériau diélectrique isolant électriquement. Les différents fils électriquement conducteurs sont contenus dans une gaine externe en matériau isolant électriquement.

Le câble électriquement conducteur 300 s'étend sur une paroi latérale 112, 113 de la rainure 110 de la plaque 100. Dans le mode de réalisation illustré à la figure 2, la plaque comprend deux câbles électriquement conducteurs 300, chaque câble 300 s'étendant le long d'une paroi latérale 112, 113 respective de la rainure. Ceci permet d'éviter la présence d'obstacle au rayonnement lumineux au droit des moyens d'illumination 200.

### 1.4. Lame transparente

La lame 400 permet de coiffer la rainure 110 formant logement pour les moyens d'illumination 200. Lorsque le dispositif d'éclairage et de chauffage comprend des moyens d'illumination 200 distribués sur les deux faces de la plaque 100, le dispositif comprend des première et deuxième lames 400 s'étendant respectivement sur les première et deuxième faces de la plaque 100, chaque lame permettant de recouvrir un logement respectif du dispositif d'éclairage et de chauffage.

Chaque lame 400 est réalisée dans un matériau transparent au rayonnement lumineux, tel qu'une résine de polysulfone. Toutefois, le matériau constituant chaque lame 400 est de préférence du verre. L'utilisation de verre permet de garantir une meilleure étanchéité du logement. Le verre présente de plus l'avantage d'être plus transparent que la résine, et de mieux résister :
- aux contraintes de températures et de pression lors des stérilisations, et
- aux traitements à base de soude (NaOH) ou d'acides (H2SO4....) utilisés lors de cycles de nettoyages du réacteur

Chaque lame 400 peut comprendre des ouvertures traversantes s'étendant au droit des trous borgnes de la plaque 100 pour permettre le passage des moyens de fixation 600.

### 1.5. Châssis et moyens de fixation

Le châssis 500 consiste en un profilé de retenue de même surface que la plaque 100. Dans le mode de réalisation illustré à la figure 2, le châssis consiste en un cadre sensiblement plan, la plaque comprenant un décrochement à la périphérie de la rainure pour permettre la réception des bords latéraux de la lame 400. Dans le mode de réalisation illustré à la figure 3, le châssis 500 diffère du mode de réalisation illustré à la figure 2 en ce que c'est lui qui comprend un décrochement sur son bord intérieur pour permettre la réception des côtés de la lame 400.

Le châssis 500 est dans un matériau peu sensible à la corrosion tel que de l'acier inoxydable.

Il est destiné à être positionné au-dessus de la lame 400 et à être boulonné sur la plaque 100 pour permettre la fixation de la lame 400. Des joints 510 en élastomère (EPDM, Viton...) peuvent être positionnés à la périphérie de la lame 400 :
- sur la face de la lame 400 destinée à venir en contact avec la plaque 100 de sorte à entourer la rainure 110 et assurer l'étanchéité entre la lame 400 et la plaque 100, et
- sur la face de la lame 400 destinée à venir en contact avec le châssis 500 de sorte à assurer l'étanchéité entre la lame 400 et le châssis 500.

Avantageusement, la plaque 100 et le châssis 500 peuvent comprendre des nervures (non représentées) pour assurer un bon ancrage de joints d'étanchéité.

Par ailleurs, le châssis 500 peut comprendre des lumières traversantes destinées à être positionnées au droit des trous borgnes de la plaque 100 et des ouvertures traversantes de la lame 400 pour le passage des moyens de fixation 600.

Ces moyens de fixation 600 peuvent consister en des boulons permettant de solidariser la plaque 100, la (ou les) lame(s) 400 et le (ou les) châssis 500. Les moyens de fixation 600 peuvent en complément comprendre un matériau de collage, ou tout autre moyen de fixation connu de l'homme du métier.

### 2. Exemple de réacteur intégrant le dispositif d'éclairage et de chauffage

### 2.1. Illustration de la génération de chaleur

En référence à la figure 5, on a illustré un exemple de réacteur mettant en œuvre la présente invention.

Le réacteur comporte principalement :
- une cuve 81 destinée à contenir une masse à traiter,
- un ensemble tournant 82 autour d'un axe Z-Z destiné à assurer un brassage de cette masse de micro-organismes, et
- une pluralité de dispositifs d'éclairage et de chauffage 83 destinés à favoriser la croissance de cette masse de micro-organismes.

La cuve comporte une paroi interne à laquelle sont fixées des dispositifs d'éclairage et de chauffage 83 dont les plans sont orientés vers l'axe de l'ensemble tournant 82 et parallèlement à celui-ci en sorte d'empêcher la formation d'un vortex au sein de la masse de micro-organismes sous l'action de l'ensemble tournant 82. Les dispositifs d'éclairage et de chauffage 83 constituent ainsi des contre-pales.

Un tel réacteur peut, en fonction de son application, comporter d'autres éléments (non représentés) tels que, notamment, une voie d'entrée d'un produit à traiter ou à dégrader au moyen de la biomasse, ou une voie d'alimentation en réactif ou en éléments nutritifs tels que des sucres pour la prolifération de la biomasse, une voie d'arrivée et de sortie d'air ou de gaz, ou une voie de soutirage de micro-organismes.

Lors de l'activation de chaque dispositif d'éclairage et de chauffage 83, les moyens d'illumination 200 génèrent un rayonnement lumineux destiné à favoriser la croissance de la masse de micro-organisme ou bien à induire la production de composés d'intérêt : en effet, la lumière peut agir sur la cellule en tant que signal pour modifier le métabolisme indépendamment de la photosynthèse. Conjointement à la génération de lumière, les moyens d'illumination 200 produisent de la chaleur. Cette chaleur est dissipée par l'intermédiaire du fond 111 de la rainure 110, celui-ci étant en contact thermique avec les moyens d'illumination 200.

Ainsi, les caractéristiques du dispositif d'éclairage et de chauffage permettent une meilleure dissipation dans le milieu culture de la chaleur générée par les moyens d'illumination 200 et une augmentation de quelques degrés du milieu de culture ou du moût de fermentation.

On a mesuré le dégagement de chaleur de deux dispositifs d'éclairage et de chauffage 83 dans un fermenteur contenant 95 Litres d'eau, sous une agitation de 300 rpm, sans débit d'air pour limiter l'évaporation durant l'expérience et garder un volume constant dans la cuve au court de l'expérience. La régulation de température de la cuve par double enveloppe n'est pas activée, la température de la pièce est contrôlée et constante aux alentours de 22°C. Le suivi de température se fait par une sonde interne avec un relevé toutes les deux minutes.

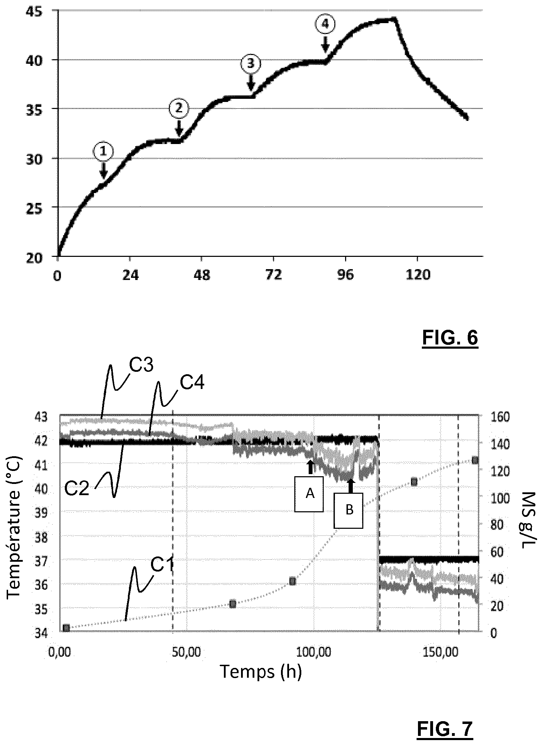

Les résultats de cette expérience sont illustrés à la figure 6. Après les premières 15 heures, la montée 1 en température de 20°C à 27°C est liée à l'agitation de l'axe de mélange.

Les dispositifs d'éclairage et de chauffage sont ensuite activés (moyens d'illumination générant un rayonnement lumineux) et la puissance augmentée par tranche de 25%, délivrant ainsi 48 watts, 96 watts, 144 watts et 192 watts respectivement, ce qui correspond à 0,48 w/L ; 0,96 w/L ; 1,44 w/L et 1,92 w/L.

Chaque palier de puissance provoque une augmentation de la température 2, 3, 4, d'environ 5°C en moins de 24h. Ce qui se traduit par une augmentation de la température de l'eau de plus de 20°C, et une température finale de 44°C pour une puissance de 1,92w/L.

Lorsque les dispositifs d'éclairage et de chauffage sont désactivés (moyens d'illumination ne générant plus de rayonnement lumineux), on observe de façon quasiment immédiate une baisse de la température de l'eau.

Outre le fait que ce système permette une dissipation de chaleur des contre-pales vers le milieu de culture afin de maintenir une température de fonctionnement acceptable pour les moyens d'illumination, il permet de faire des économies d'énergie lorsque les cultures de micro-organismes comme les algues nécessitent également de chauffer le milieu de culture.

Comme exemple nous pouvons citer des souches comme *Galderia sulphuraria* dont l'optimum de croissance se situe entre 42°C et 45°C. L'augmentation de la température du milieu de culture est dans ce cas un avantage.

Le graphique de la figure 7 illustre l'effet du dégagement de chaleur du dispositif d'éclairage et de chauffage lors de la croissance de *Galderia sulphuraria* dans un bioréacteur incluant une double-paroi dans laquelle circule un fluide caloporteur (ici de l'eau) permettant de maintenir le milieu de culture de *Galderia sulphuraria* à une température désirée.

Ce graphique comprend :
- Une première courbe C1 représentative de la croissance de la souche *Galderia sulphuraria* exprimé en g de matière sèche par litre de culture,
- Une deuxième courbe C2 correspondant à l'enregistrement de la température du milieu de culture à l'intérieur du réacteur, la température du milieu de culture étant maintenue constante à 42°C jusqu'au temps 125h, puis la température du milieu de culture étant volontairement abaissée à 37°C par la suite jusqu'à la fin de la culture,
- Une troisième courbe C3 correspondant à l'enregistrement au cours du temps de la température d'entrée du fluide caloporteur servant à réguler la température du milieu de culture, et
- Une quatrième courbe C4 illustrant la température de sortie du fluide caloporteur.

A 100 heures de fermentation (point A) les dispositifs d'éclairage et de chauffage sont activés. La température de la culture à l'intérieur de la cuve ne fluctue pas et reste stable à 42°C alors que les températures d'entrée et de sortie du fluide caloporteur contenu dans la double enveloppe diminuent de plusieurs degrés en quelques heures. Ces enregistrements traduisent bien l'effet de diffusion de la chaleur générée par les moyens d'illumination vers le milieu de culture via la structure métallique du dispositif d'éclairage et de chauffage.

De façon réversible, lorsque l'on désactive les dispositifs d'éclairage et de chauffage (point B à environ 115 h) les températures d'entrée et de sortie du fluide caloporteur augmentent, traduisant la nécessité de chauffer ce fluide caloporteur pour maintenir constante la température du milieu de culture.

A 125 heures, la température du milieu de culture été volontairement abaissée à 37°C, et les dispositifs d'éclairage et de chauffage ont été réactivés. De façon similaire à ce qui a été observé à 42°C, on observe une diminution progressive de la température du fluide caloporteur contenu dans la double enveloppe.

La puissance lumineuse utilisée lors de cette expérience est de 2w/L, et une température ambiante de 22°C. Il est donc envisageable de pouvoir très largement augmenter la puissance des moyens d'illumination des dispositifs d'éclairage et de chauffage, à 4w/l voir même jusqu'à 6w/l par exemple sans que cela ne fasse intervenir un refroidissement du fluide caloporteur contenu dans la double enveloppe.

La figure 7 illustre démontre que sans dispositif d'éclairage et de chauffage, le fluide caloporteur contenu dans la double paroi du réacteur (et qui permet de réguler la température du milieu de culture) doit être chauffé en permanence pour maintenir la température du milieu de culture à une valeur de consigne.

### 3. Conclusions

Le dispositif d'éclairage et de chauffage décrit précédemment permet d'augmenter la quantité de lumière délivrée à une masse à traiter. Ceci est valable pour des cultures en mixotrophie, en autotrophie sur des organismes photosynthétiques mais également pour des cultures en mixotrophie à dominante hétérotrophe où la lumière n'est pas importante pour l'activité photosynthétique mais par exemple pour l'induction de molécules d'intérêt comme des pigments (WO2017050917), et/ou de l'huile.

Ce dispositif permet également d'augmenter la température du milieu de culture.

Le lecteur aura compris que de nombreuses modifications peuvent être apportées à l'invention décrite précédemment sans sortir matériellement des nouveaux enseignements et des avantages décrits ici.

Par exemple, dans la description qui précède, le dispositif d'éclairage et de chauffage était intégré dans un réacteur incluant un ensemble tournant destiné à assurer un brassage de cette masse de micro-organismes. Il est bien évident pour l'homme du métier que le dispositif d'éclairage et de chauffage décrit précédemment pourrait être intégré dans un réacteur dépourvu d'ensemble tournant.

Par conséquent, toutes les modifications de ce type sont destinées à être incorporées à l'intérieur de la portée des revendications jointes.

## Revendications

1. Réacteur incluant une cuve (81) destinée à contenir une masse à traiter et au moins un dispositif d'éclairage et de chauffage (83) destiné à favoriser le traitement de cette masse, ***caractérisé en ce que*** le dispositif d'éclairage et de chauffage comprend :
- une plaque (100) incluant au moins une rainure (110) s'étendant longitudinalement et composée d'un fond (111) et de deux parois latérales (112, 113),
- des moyens d'illumination (200) pour générer un rayonnement lumineux, lesdits moyens d'illumination (200) étant disposés dans la rainure (110) et étant en contact thermique avec le fond (111) de la rainure (110), de sorte que la chaleur générée par les moyens d'illumination est transmise à la masse à traiter par l'intermédiaire du fond de la plaque.

2. Réacteur selon la revendication 1, ***dans lequel*** le dispositif d'éclairage et de chauffage comprend :
- au moins un câble électriquement conducteur (300) pour connecter électriquement les moyens d'illumination (200) à une source en alimentation électrique distante, le câble (300) s'étendant le long d'une des parois latérales (112, 113) de la rainure (110),
- au moins une lame (400) transparente au rayonnement lumineux s'étendant sur la plaque (100) pour coiffer la rainure (110).

3. Réacteur selon l'une quelconque des revendications 1 ou 2, ***dans lequel*** les moyens d'illumination (200) comprennent au moins un module de diodes électroluminescentes à montage direct de puces.

4. Réacteur selon la revendication 2, ***dans lequel*** le matériau constituant la lame (400) est du verre.

5. Réacteur selon l'une quelconque des revendications précédentes, ***dans lequel*** chaque plaque (100) comprend au moins deux panonceaux démontables reliés bord à bord de sorte à s'étendre dans un même plan pour constituer la plaque (100).

6. Réacteur selon la revendication 5, ***dans lequel*** les moyens d'illumination (200) d'un panonceau sont activables indépendamment de l'activation des moyens d'illumination (200) des autres panonceaux.

7. Réacteur selon l'une quelconque des revendications précédentes, ***lequel*** comprend en outre un agitateur (82) tournant autour d'un axe Z pour brasser la masse à traiter, chaque dispositif d'éclairage et de chauffage (83) étant fixé à une face interne de la cuve et s'étendant dans un plan orienté vers l'axe de l'agitateur (82) pour former une contre-pâle permettant d'empêcher la formation d'un vortex au sein de la masse à traiter.

8. Réacteur selon l'une quelconque des revendications précédentes, ***dans lequel*** le dispositif d'éclairage et de chauffage est agencé de sorte que la dissipation de chaleur permet d'augmenter la température du milieu de culture d'au moins 2 degrés Celsius et facilite ainsi la croissance des microorganismes.

## Patentansprüche

1. Reaktor, der einen Tank (81), der dazu bestimmt ist, eine zu behandelnde Masse zu enthalten, und mindestens eine Beleuchtungs- und Heizvorrichtung (83) umfasst, die dazu bestimmt ist, die Behandlung dieser Masse zu begünstigen, **dadurch gekennzeichnet, dass** die Beleuchtungs- und Heizvorrichtung umfasst:
- eine Platte (100), die mindestens eine Rinne (110) umfasst, die sich in Längsrichtung erstreckt und aus einem Boden (111) und aus zwei Seitenwänden (112, 113) besteht,
- Beleuchtungsmittel (200) zum Erzeugen einer Lichtstrahlung, wobei die Beleuchtungsmittel (200) in der Rinne (110) angeordnet sind und in thermischem Kontakt mit dem Boden (111) der Rinne (110) stehen, derart dass die von den Beleuchtungsmitteln erzeugte Wärme über den Boden der Platte auf die zu behandelnde Masse übertragen wird.

2. Reaktor nach Anspruch 1, wobei die Beleuchtungs- und Heizvorrichtung umfasst:
- mindestens ein elektrisch leitfähiges Kabel (300), um die Beleuchtungsmittel (200) elektrisch mit einer entfernten elektrischen Versorgungsquelle zu verbinden, wobei das Kabel (300) sich entlang einer der seitlichen Wände (112, 113) der Rinne (110) erstreckt,
- mindestens ein gegenüber der Lichtstrahlung durchlässiges Blatt (400), das sich auf der Platte (100) erstreckt, um die Rinne (110) zu bedecken.

3. Reaktor nach einem der Ansprüche 1 oder 2, wobei die Beleuchtungsmittel (200) mindestens ein Leuchtdiodenmodul mit direkter Chipmontage umfassen.

4. Reaktor nach Anspruch 2, wobei das Material, das das Blatt (400) bildet, Glas ist.

5. Reaktor nach einem der vorhergehenden Ansprüche, wobei jede Platte (100) mindestens zwei demontierbare Schilder umfasst, die derart stumpf verbunden sind, dass sie sich in einer selben Ebene erstrecken, um die Platte (100) zu bilden.

6. Reaktor nach Anspruch 5, wobei die Beleuchtungsmittel (200) eines Schildes unabhängig von der Aktivierung der Beleuchtungsmittel (200) der anderen Schilder aktivierbar sind.

7. Reaktor nach einem der vorhergehenden Ansprüche, der ferner ein Rührwerk (82) umfasst, das sich um eine Achse Z dreht, um die zu behandelnde Masse umzurühren, wobei jede Beleuchtungs- und Heizvorrichtung (83) an einer Innenfläche des Tanks befestigt ist und sich in einer Ebene erstreckt, die hin zur Achse des Rührwerks (82) ausgerichtet ist, um einen Gegenflügel zu bilden, der die Bildung eines Wirbels im Inneren der zu behandelnden Masse verhindert.

8. Reaktor nach einem der vorhergehenden Ansprüche, wobei die Beleuchtungs- und Heizvorrichtung derart gestaltet ist, dass die Wärmestreuung das Erhöhen der Temperatur des Kulturmediums um mindestens 2 Grad Celsius ermöglicht und somit das Wachstum der Mikroorganismen erleichtert.

## Claims

1. A reactor including a tank (81) intended to contain a mass to be treated and at least one lighting and heating device (83) intended to promote the treatment of this mass, **characterized in that** the lighting and heating device comprises:
- a plate (100) including at least one groove (110) extending longitudinally and composed of a bottom (111) and two side walls (112, 113),
- illumination means (200) for generating a light radiation, said illumination means (200) being disposed in the groove (110) and being in thermal contact with the bottom (111) of the groove (110), so that the heat generated by the illumination means is transmitted to the mass to be treated via the bottom of the plate.

2. The reactor as claimed in claim 1, wherein the lighting and heating device comprises:
- at least one electrically conductive cable (300) to electrically connect the illumination means (200) to a remote electrical power source, the cable (300) extending along one of the side walls (112, 113) of the groove (110),
- at least one blade (400) transparent to light radiation extending over the plate (100) to cover the groove (110).

3. The reactor as claimed in any one of claims 1 or 2, wherein the illumination means (200) comprise at least one module of chip-on-board light emitting diodes.

4. The reactor as claimed in claim 2, wherein the material constituting the blade (400) is glass.

5. The reactor as claimed in any one of the preceding claims, wherein each plate (100) comprises at least two removable panels joined edge to edge so as to extend in the same plane to constitute the plate (100).

6. The reactor as claimed in claim 5, wherein the illumination means (200) of one panel can be activated independently of the activation of the illumination means (200) of the other panels.

7. The reactor as claimed in any one of the preceding claims, which further comprises an agitator (82) rotating about an axis Z to stir the mass to be treated, each lighting and heating device (83) being attached to an inner face of the tank and extending in a plane oriented toward the axis of the agitator (82) to form a counter-blade for preventing the formation of a vortex within the mass to be treated.

8. The reactor as claimed in any one of the preceding claims, wherein the lighting and heating device is arranged so that the heat dissipation allows the temperature of the culture medium to be increased by at least 2 degrees Celsius and thereby facilitates the growth of the microorganisms.
